# EUROPEAN PATENT SPECIFICATION

(11) **EP 1 617 898 B1**
(45) Date of publication and mention of the grant of the patent: **13.08.2008**
(21) Application number: 04749976.9
(22) Date of filing: 09.04.2004
(51) Int. Cl.: A61N 1/362

(54) **PACEMAKER WITH IMPROVED CAPABILITY FOR DETECTING ONSET OF TACHYARRHYTHMIAS AND HEART FAILURE**
HERZSCHRITTMACHER MIT VERBESSERTER FÄHIGKEIT FÜR DAS ERMITTELN DES ANGRIFFES VON TACHYARRHYTHMIAS UND VON HERZVERSAGEN
STIMULATEUR CARDIAQUE A FONCTION AMELIOREE PERMETTANT DE DETECTER L'APPARITION DE TACHYARYTHMIES ET D'UNE INSUFFISANCE CARDIAQUE

(30) Priority: 24.04.2003 US 422712
(43) Date of publication of application: 25.01.2006
(73) Proprietor: MEDTRONIC, INC., Minneapolis, MN 55432 (US)
(72) Inventor: VAN DAM, Peter, M., NL-6981 EE Doesburg (NL); VAN OOSTEROM, Adriaan, NL-5439NL Linden (NL)
(74) Representative: Hughes, Andrea Michelle
(86) International application number: PCT/US2004/011137
(87) International publication number: WO 2004/096350

(56) References cited:
- EP-A- 0 469 817
- US-A- 5 871 511

## Description

This invention relates to pacing systems that incorporate a diagnostic capability for monitoring cardiac conditions and providing a response to a detected abnormal condition and, more particularly, to implantable pacing systems for monitoring cardiac conditions and sensing the onset of atrial tachyarrhythmias or heart failure, and that provide a pacing therapy in response to such sensing.

Previously, the treatment of abnormal heart rhythms, or cardiac arrhythmias, has received a great deal of attention and has been the subject of much investigation and medical literature. One of the most common such abnormal heart rhythm is atrial fibrillation, also referred to as AF, which affects over 2 million persons every year. AF is characterized by an uncontrolled high rate shivering or quivering of the atria, with the atria fibrillating at 300 to 600 times per minute. During AF there is a loss of synchrony between the atria and the ventricles, resulting in substantial failure of pumping from the atria to the ventricles. The passage of excitation impulses through the AV node to the ventricles is irregular and at an abnormally high rate, resulting in irregular ventricular rates on the order of 150 beats per minute and above. Consequently, when AF becomes symptomatic, the patient feels shortness of breath and dizziness, reflecting the failure of good pumping function.

AF presents more serious consequences to the patient than just the symptoms, which themselves can be debilitating. During AF the blood is not efficiently flushed from the heart's chambers, resulting in pooling and a greater likelihood of clotting. In about 5% of AF cases, such clots are discharged from the heart and produce a stroke; the American Heart Association estimates that AF causes over 70,000 strokes annually. And AF can lead to other problems, including cardiomyopathy and congestive heart failure (CHF). AF can occur directly, or it can occur as a secondary event initiated from other arrhythmias such as atrial tachycardia (AT) or atrial flutter.

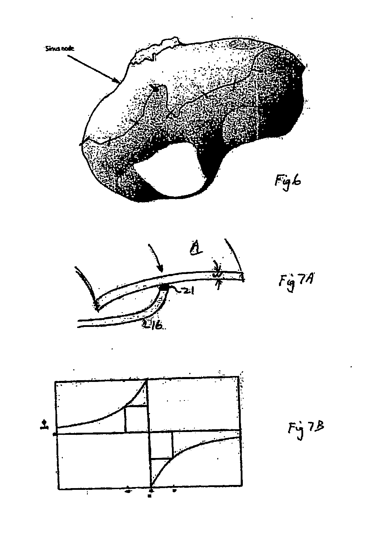

The natural activation of the atrial myocardium originates from the sinus node and travels throughout the entire atrium, causing contraction. Figure 6 illustrates the position of an activation, or depolarization wavefront traveling through atria. Since the atrial wall is rather thin, about 2 mm, this means that approximately all the myocardial cells through the atrial wall at the wavefront are activated simultaneously. A tip electrode positioned against the atrial wall senses when a depolarization wave travels past the electrode, such that the intracardial P-wave is a local phenomenon. The repolarization signal is too weak to be normally seen, such that the P-wave that can be sensed by such a pacemaker lead is a signature of the depolarization wave as it passes the electrode. The maximum amplitude and "width" of the P-wave portion of the IECG signal are a function of the velocity of the activation front as it passes the electrode, and the smaller the electrode the greater the spike of the sensed signal.

The medical literature shows that the atria are vulnerable to AT or AF when the atrial wall is characterized by non-uniformity of conduction and by slow conduction, giving rise to reentry circuits or pathways. Slow impulse conduction can give rise to reentrant arrhythmias by reducing the effective wavelength of the reentering wavefront, such that the available reentry pathway can accommodate it. Stating the matter differently, a slower conduction enables a return or circular path with the proper timing such that when the wavefront has made a loop the initially depolarized cells have repolarized and are no longer refractory. In such a case, the cells can be depolarized by the reentry wave, triggering a continuing impulse wavefront without receiving an impulse from the sinus node. A number of factors can determine the velocity at which an action potential, or impulse is propagated. Among these are the fast inward sodium current during the upstroke of the action potential and the axial resistance (degree of cellular coupling) to the flow of the local current through the myocardial fibres. The conduction property of cardiac muscle is anisotropic in that it conducts longitudinally axially, or longitudinally better than it does transversely. A healthy heart has uniform anisotropy, such that the advancing wavefront is smooth in all directions. A heart characterized by non-uniform anisotropy is one where the wavefront does not advance smoothly, and includes areas of unidirectional block and return conduction pathways. It is this non-uniform anisotropy, together with slower conduction, that can produce an arrhythmogenic substrate and make the heart vulnerable to AF.

By monitoring the effective conduction velocity of the myocardium, the development of an arrhythmogenic substrate can be determined, enabling an indication of the onset of AF or another arrhythmia. When the velocity drops, this indicates either a slowing of conduction or a widening (thickening) of the heart wall. In the case of the atria, the heart wall retains a rather constant thickness, so a sensed decrease in conduction velocity indicates a slowing of conduction. When the conduction velocity is determined to have dropped to a certain threshold, or a given amount below the patient's normal velocity, this is an indication of conditions that give rise to AF.

Our studies of conduction in the human heart wall show that the waveform of an impulse that propagates through cardiac tissue can provide information concerning conduction velocity, given that the wall thickness is constant. The conduction velocity and wave width are closely related for an atrial activation wave. In an atrium, the wall is rather thin, being on average about 2mm in thickness. The activation wave in a normal atrium takes about 125 ms, i.e., this is the time from initiation of the wave at the sinus node to finished activation of the excitable atrial muscle. Assuming no change in wall thickness, a change in the speed of passage of the atrial waveform is an indication of a change in effective conduction velocity, and vice versa.

We have examined the effect of wall thickness on an IEGM waveform measured on the tip electrode 21 of a lead positioned against a heart wall 8 as illustrated in Figure 7A. We assume that the impulse wave passes perpendicularly to the tip, and that the impulse wave is a flat wave with a width w (corresponding to the wall thickness), as shown in Figure 7A. For this situation, the theoretical sensed waveform at the tip located at x(o) is as shown in Figure 7B. It is noted that the potential at the tip is only half the maximum value when the excitation wave is a distance w away from the tip. From this we conclude that for constant propagation velocity, a wider wall thickness will induce a wider waveform (longer pulse width in terms of time, Δt) in an electrode placed against the heart wall.

Our studies have demonstrated that in the atria, wall thickness is substantially uniform. However, this is not the case for the left ventricle of a patient who is progressing toward heart failure (HF), commonly also referred to as congestive heart failure (CHF). In CHF, a first response of the heart to increased load is to increase its contraction power, i.e., ability to pump more blood. In many cases this means that the heart wall becomes thicker, or hypertrophied. When this happens, the stroke volume reduces, which eventually means that the patient becomes short of energy. The last stage of CHF can be a dilated heart, a condition that occurs due to apoptosis, i.e., cell death. In this condition, the heart wall becomes thinner, with even more decreased pumping strength. The left ventricle is the chamber that pumps blood into the body, and therefore the one that must be monitored for changes in wall thickness. Assuming that conduction velocity throughout the left ventricle is substantially constant at onset of CHF and through its progression, the wall thickness can be detected by an electrode positioned just beneath the left free wall. The greater the width (duration) of the sensed signal from passage of the activation wave (the R wave), the greater the wall thickness, and vice versa.

The pacing art has produced a number of pacing therapies that are available to aid in suppressing AF or alleviating the symptoms of AF. Thus, pacemaker systems have incorporated therapies that respond to high atrial rates, designed to treat already developed arrhythmias. However, what is needed is a better means for early detection of the onset of AF. Such early detection or prediction of AF can be accomplished by getting an accurate picture of a developing arrhythrnic substrate, which in turn can be obtained from sensed waveform data. In U.S. Patent No. 5,184,615 to Nappholz, the pacemaker obtains a paced depolarization integral (PDI) that is the time integral of the depolarization voltage amplitude over the interval of the QRS complex. This integral is analyzed over time, to detect changes that might indicate cardiac arrhythmias. However, the integral is not an accurate indication of conduction changes; and a measure of a QRS waveform in response to a pacing pulse does not provide any predictability of onset of AF. The approach taken in U.S. Patent No. 5.243,981 is an improvement, but still has problems. In that patent, two spaced electrodes are positioned against the heart wall and coupled to respective amplifiers to detect the relative arrival times of the depolarization wavefront at the electrodes. The difference in arrival times is used to determine a measure of conduction velocity. However, such arrival times do not contain information concerning the overall non-uniform anisotropy of the atrial wall, or the degree to which there is slowing of conduction in reentry paths that can be a sign of AF. Depending on how the wave has traveled, the excitation wave can arrive at the two points at much the same time, or with a time difference that doesn't accurately reflect the overall conduction scheme. Since the conduction changes can occur anywhere throughout the entire atria, the measurement must represent the entire wave, not just the leading edge of the wave at several points.

Publications that explain the mechanism of conduction in cardiac muscle and refer to conduction velocity include Cellular Electrophysiology, Part III, page 148; Arrhythrnia Mecha, Part VIII, pp 490, 512 and 548-550; and Circulation, Chow et. al, Wavefront Propagation in Circuits Causing Human VT, pp 2172-2178, May 2002. The following patents disclose implantable pacing systems and refer directly or indirectly to cardiac conduction velocity.

### Table 1

Patent Number Inventor(s) Issue Date
5,184,61 Nappholz et. a. Feb. 9, 1993
5,243,981 Hudrlik Sept. 14, 1993
5,267,560 Cohen Dec. 7, 1993

In the device of US patent No, 5312441, antitachycardie therapy is delivered when the wicht of the patients R-wave is greaten those a minum width threshold.

As those of ordinary skill in the art will appreciate readily upon reading the Summary of the Invention, Detailed Description of the Preferred Embodiments and Claims set forth below, many of the devices and methods disclosed in the patents of Table 1 may be modified advantageously by using the teachings of the present invention.

It is an object of the invention to provide a system for obtaining information from IECG signals and deriving therefrom data that represents a measure of overall atrial conduction velocity, and for processing the velocity data to obtain an indication of whether or not the heart is at or near AF.

It is another object of this invention to provide an implantable pacing system with monitoring capability to obtain data indicative of conduction velocity throughout a patient's atria, and with an algorithm for processing such data to provide a determination of whether the patient is at or near AF.

It is another object of this invention to provide a system for obtaining IECG data from within a patient's atrium and for utilizing this data to provide an indication of whether the patient is at onset of AF.

It is another object of this invention to provide a system for determining wall thickness in the left free ventricular wall. It is another object of this invention to provide a pacing system for diagnosing ventricular wall thickness based on variations in the duration of the excitation wave as it passes a location in the left free ventricular wall and utilizing such diagnosed wall thickness to obtain an indication of CHF.

It is another object of this invention to provide a system for determining conduction velocity in a patient's atria by utilizing a tip electrode that can be placed substantially anywhere against the wall of an atrial chamber of the patient in order to obtain local IECG signals that contain information about atrial conduction velocity. The invention is as defined in the independent claim

In a first embodiment, there is provided an implantable pacing system that includes a lead having at least a tip electrode adapted to be positioned within the patient's right atrium against the cardiac wall. The sensed P-wave signals from the patient's atria are processed to obtain measures of overall atrial conduction velocity, and such measures are processed with an algorithm to provide a prediction of AF. In a preferred embodiment; the P-wave signals are processed periodically to provide an average relative velocity measure, and the measure is compared with prior stored velocity data to determine whether conduction velocity has slowed such that the patient is likely at or near AF. When and if there is an indication of the onset of AF, a suitable pacing therapy is applied.

In the use of the first embodiment, a lead with a tip electrode is inserted into the patient's right atrium and the tip electrode is positioned adjacent the atrial wall. The exact placement is not critical so long as the tip is snug with the wall muscle. Sensing of P-waves can be done either unilaterally, with the pacemaker can used as the other electrode, or bilaterally with a suitable ring electrode placed sufficiently proximal to the tip electrode so that the sensed signal is much the same as with the unilateral embodiment. The P-waves are analyzed to determine their time width (duration), and each width determination in milliseconds is compared with prior values and stored. When the comparison indicates onset of AF, the pacing system is controlled to apply a suitable pacing therapy.

In a second embodiment of the invention, a lead is placed against or near the left ventricular free wall, e.g., within the left main coronary artery. QRS signals are obtained from the tip electrode of the lead, and width data of each such waveform is obtained. Another lead may be placed suitably in the right ventricle for obtaining reference waveforms passing a tip electrode at the end of the lead in the right ventricle, to indicate substantially constant ventricular conduction velocity. The system algorithm determines when the depolarization waves sensed in the left ventricle are stable, and when this situation is present analyzes the data from the ventricular free wall to obtain a measure of ventricular wall thickness. The ventricular wall thickness data in turn is processed to determine the degree, if any, of ventricular wall hypertrophy and CHF.

In another embodiment, plural leads are utilized as described above in a multi-chamber system to obtain predictions of AF, VF and/or HF.

Preferred embodiments will now be described, by way of example only, with reference to the drawings.
Figure 1 shows a simplified schematic view of one embodiment of an implantable medical device that can be employed in the present invention.
Figure 2 shows a graphic representation of an implantable medical device interconnected with a human or mammalian heart, illustrating the device connector portion and the leads between the device and the heart.
Figure 3 shows a functional schematic diagram showing the primary constituent components of an implantable medical device in accordance with an embodiment of this invention.
Figure 4 shows a graphic representation of an embodiment of this invention showing an implantable PCD device interconnected with a heart, the system of this embodiment providing pacing, cardio version and defibrillation.
Figure 5 is a functional schematic diagram of an implantable PCD embodiment of this invention.
Figure 6 shows a schematic sketch of atria, with a jagged line that illustrates an advancing excitation wavefront.
Figure 7A is a sketch that shows a depolarization wavefront passing through a heart wall with a thickness w, with a tip electrode positioned normal to the wall and the passing wavefront.
Figure 7B is a graph of sensed potential vs. distance obtained from a tip electrode placed under a heart wall such that the depolarization impulse travels perpendicularly past the tip.
Figure 8 is an overall flow diagram illustrating the collection of P-wave data from a tip electrode positioned against the atrial wall and the processing of such data for determining when there is an indication of the onset of AF.
Figure 9 is an expanded sub-routine of the steps of block 202 in Figure 8, for checking sensed waveforms and obtaining values of wave width (representing relative velocity in Figure 8).
Figure 10 is an expanded flow diagram of block 204 in Figure 8, for obtaining measures of average velocity and threshold velocity.
Figure 11A is a schematic drawing that shows a tip electrode positioned on the lateral wall of the left ventricle.
Figure 11B is a flow diagram illustrating an algorithm for determining wall thickness in the free left ventricular wall in accordance with this invention.
Figure 11C is a flow diagram illustrating a modification of the algorithm of Figure 11B by using right ventricular signals as reference signals for checking for stability of ventricular conduction.

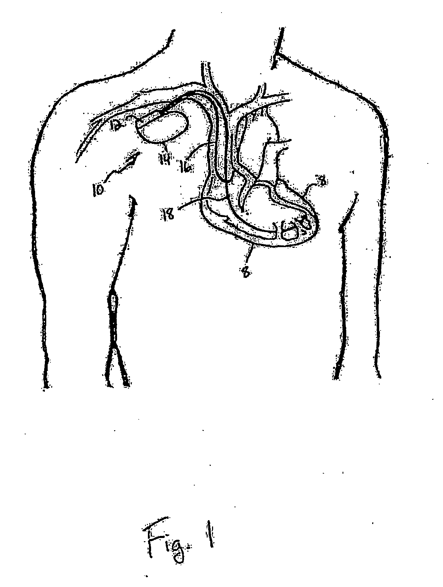

The invention may be embodied in many forms, as the following specification and Figures illustrate. Figure 1 is a simplified schematic view of one embodiment of implantable medical device ("IMD") 10 of the present invention. IMD 10 shown in Figure 1 is a pacemaker comprising at least one of pacing and sensing leads 16 and 18 attached to hermetically sealed enclosure 14 and implanted near human or mammalian heart 8. Pacing and sensing leads 16 and 18 sense electrical signals attendant to the depolarization and re-polarization of the heart 8, and further provide pacing pulses for causing depolarization of cardiac tissue in the vicinity of the distal ends thereof. Leads 16 and 18 may have unipolar or bipolar electrodes disposed thereon, as is well known in the art. In the practice of this invention, as discussed further below, a unipolar type lead with a tip electrode is preferred. However, a lead with a tip electrode and a second electrode placed substantially proximal so as to get substantially equivalent unipolar tip sensing can also be used Examples of IMD 10 include implantable cardiac pacemakers disclosed in U.S. Patent No. 5,158,078 to Bennett et al., U.S. Patent No. 5,312,453 to Shelton et al. or U.S. Patent No. 5,144,949 to Olson.

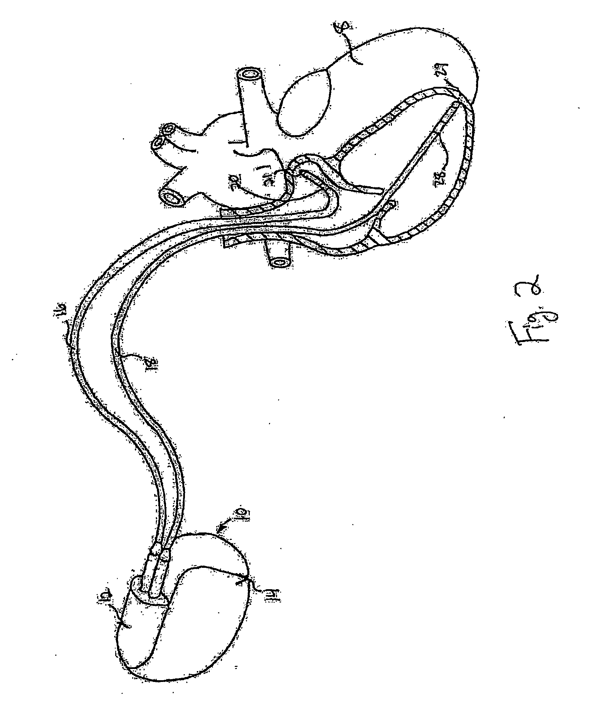

Figure 2 shows connector module 12 and hermetically sealed enclosure 14 of IMD 10 located in and near human or mammalian heart 8. Atrial and ventricular pacing leads 16 and 18 extend from connector header module 12 to the right atrium and ventricle, respectively, of heart 8. Atrial electrodes 20 and 21 disposed at the distal end of atrial pacing lead 16 are located in the right atrium. Ventricular electrodes 28 and 29 at the distal end of ventricular pacing lead 18 are located in the right ventricle.

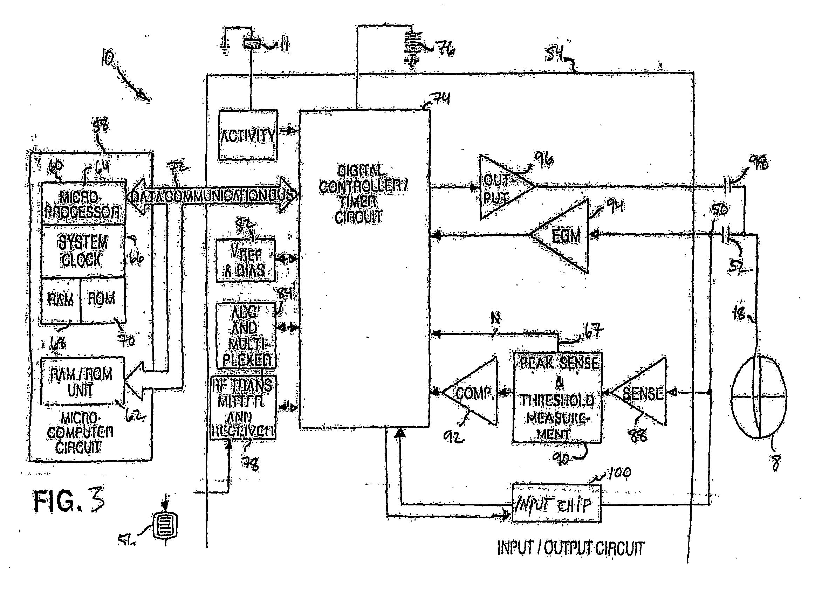

Figure 3 shows a block diagram illustrating the constituent components of IMD 10 in accordance with one embodiment of the present invention, where IMD 10 is pacemaker having a microprocessor-based architecture. IMD 10 is shown as including activity sensor or accelerometer 11, which is preferably a piezoceramic accelerometer bonded to a hybrid circuit located inside enclosure 14. Activity sensor 11 typically (although not necessarily) provides a sensor output that varies as a function of a measured parameter relating to a patient's metabolic requirements. For the sake of convenience, IMD 10 in Figure 3 is shown with lead 18 only connected thereto; similar circuitry and connections not explicitly shown in Figure 3 apply to lead 16.

IMD 10 in Figure 3 is most preferably programmable by means of an external programming unit (not shown in the Figures). One such programmer is the commercially available Medtronic Model 9790 programmer, which is microprocessor-based and provides a series of encoded signals to IMD 10, typically through a programming head which transmits or telemeters radio-frequency (RF) encoded signals to IMD 10. Such a telemetry system is described in U.S. Patent No. 5,312,453 to Wyborny et al.

The programming methodology disclosed in Wyborny et al.'s '453 patent is identified herein for illustrative purposes only. Any of a number of suitable programming and telemetry methodologies known in the art may be employed so long as the desired information is transmitted to and from the pacemaker.

As shown in Figure 3, lead 18 is coupled to node 50 in IMD 10 through input capacitor 52. Activity sensor or accelerometer 11 is most preferably attached to a hybrid circuit located inside hermetically sealed enclosure 14 of IMD 10. The output signal provided by activity sensor 11 is coupled to input/output circuit 54. Input/output circuit 54 contains analog circuits for interfacing to heart 8, activity sensor 11, antenna 56 and circuits for the application of stimulating pulses to heart 8. The rate of heart 8 is controlled by software-implemented algorithms stored in microcomputer circuit 58.

Microcomputer circuit 58 preferably comprises on-board circuit 60 and off-board circuit 62. Circuit 58 may correspond to a microcomputer circuit disclosed in U.S. Patent No. 5,312,453 to Shelton et al.. On-board circuit 60 preferably includes microprocessor 64, system clock circuit 66 and on-board RAM 68 and ROM 70. Off-board circuit 62 preferably comprises a RAM/ROM unit. On-board circuit 60 and off-board circuit 62 are each coupled by data communication bus 72 to digital controller/timer circuit 74. Microcomputer circuit 58 may comprise a custom integrated circuit device augmented by standard RAM/ROM components.

Electrical components shown in Figure 3 are powered by an appropriate implantable battery power source 76 in accordance with common practice in the art. For the sake of clarity, the coupling of battery power to the various components of IMD 10 is not shown in the Figures. Antenna 56 is connected to input/output circuit 54 to permit uplink/downlink telemetry through RF transmitter and receiver telemetry unit 78. By way of example, telemetry unit 78 may correspond to that disclosed in U.S. Patent No. 4,566,063 issued to Thompson et al., or to that disclosed in the above-referenced '453 patent to Wyborny et al. It is generally preferred that the particular programming and telemetry scheme selected permit the entry and storage of cardiac rate-response parameters. The specific embodiments of antenna 56, input/output circuit 54 and telemetry unit 78 presented herein are shown for illustrative purposes only, and are not intended to limit the scope of the present invention.

Continuing to refer to Figure 3, V_{REF} and Bias circuit 82 most preferably generates stable voltage reference and bias currents for analog circuits included in input/output circuit 54. Analog-to-digital converter (ADC) and multiplexer unit 84 digitizes analog signals and voltages to provide "real-time" telemetry intracardiac signals and battery end-of-life (EOL) replacement functions. Operating commands for controlling the timing of IMD 10 are coupled by data bus 72 to digital controller/timer circuit 74, where digital timers and counters establish the overall escape interval of the IMD 10 as well as various refractory, blanking and other timing windows for controlling the operation of peripheral components disposed within input/output circuit 54.

Digital controller/timer circuit 74 is preferably coupled to sensing circuitry, including sense amplifier 88, peak sense and threshold measurement unit 90 and comparator/threshold detector 92. Circuit 74 is further preferably coupled to electrogram (EGM) amplifier 94 for receiving amplified and processed signals sensed by lead 18. Sense amplifier 88 amplifies sensed electrical cardiac signals and provides an amplified signal to peak sense and threshold measurement circuitry 90, which in turn provides an indication of peak sensed voltages and measured sense amplifier threshold voltages on multiple conductor signal path 67 to digital controller/timer circuit 74. An amplified sense amplifier signal is then provided to comparator/threshold detector 92. By way of example, sense amplifier 88 may correspond to that disclosed in U.S. Patent No. 4,379,459 to Stein.

The electrogram signal provided by EGM amplifier 94 is employed when IMD 10 is being interrogated by an external programmer to transmit a representation of a cardiac analog electrogram. See, for example, U.S. Patent No. 4,556,063 to Thompson et al.

Output pulse generator 96 provides pacing stimuli to patient's heart 8 through coupling capacitor 98 in response to a pacing trigger signal provided by digital controller/timer circuit 74 each time the escape interval times out, an externally transmitted pacing command is received or in response to other stored commands as is well known in the pacing art. By way of example, output amplifier 96 may correspond generally to an output amplifier disclosed in U.S. Patent No. 4,476,868 to Thompson.

The specific embodiments of input amplifier 88, output amplifier 96 and EGM amplifier 94 identified herein are presented for illustrative purposes only, and are not intended to be limiting in respect of the scope of the present invention. In another embodiment, signals from the patient's heart are coupled to an input channel chip shown at 100, which chip provides outputs to the controller 74. The preferred embodiment of this chip incorporates DSP circuitry as is further disclosed in U.S. Patent No. 6,029,087. The specific embodiments of such circuits may not be critical to practicing some embodiments of the present invention so long as they provide means for generating a stimulating pulse and are capable of providing signals indicative of natural or stimulated contractions of heart 8. In some preferred embodiments of the present invention, IMD 10 may operate in various non-rate-responsive modes, including, but not limited to, DDD, DDI, VVI, VOO and VVT modes. In other preferred embodiments of the present invention, IMD 10 may operate in various rate-responsive, including, but not limited to, DDDR, DDIR, VVIR, VOOR and VVTR modes. Some embodiments of the present invention are capable of operating in both non-rate-responsive and rate responsive modes. Moreover, in various embodiments of the present invention IMD 10 may be programmably configured to operate so that it varies the rate at which it delivers stimulating pulses to heart 8 only in response to one or more selected sensor outputs being generated. Numerous pacemaker features and functions not explicitly mentioned herein may be incorporated into IMD 10 while remaining within the scope of the present invention.

The present invention is not limited in scope to single-sensor or dual-sensor pacemakers, and is not limited to IMD's comprising activity or pressure sensors only. Nor is the present invention limited in scope to single-chamber pacemakers, single-chamber leads for pacemakers or single-sensor or dual-sensor leads for pacemakers. Thus, various embodiments of the present invention may be practiced in conjunction with more than two leads or with multiple-chamber pacemakers, for example. At least some embodiments of the present invention may be applied equally well in the contexts of single-, dual-, triple- or quadruple- chamber pacemakers or other types of IMD's. See, for example, U.S. Patent No. 5,800,465 to Thompson et al.

IMD 10 may also be a pacemaker-cardioverter- defibrillator ("PCD") corresponding to any of numerous commercially available implantable PCD's. Various embodiments of the present invention may be practiced in conjunction with PCD's such as those disclosed in U.S. Patent No. 5,545,186 to Olson et al., U.S. Patent No. 5,354,316 to Keimel, U.S. Patent No. 5,314,430 to Bardy, U.S. Patent No. 5,131,388 to Pless and U.S. Patent No. 4,821,723 to Baker et al.

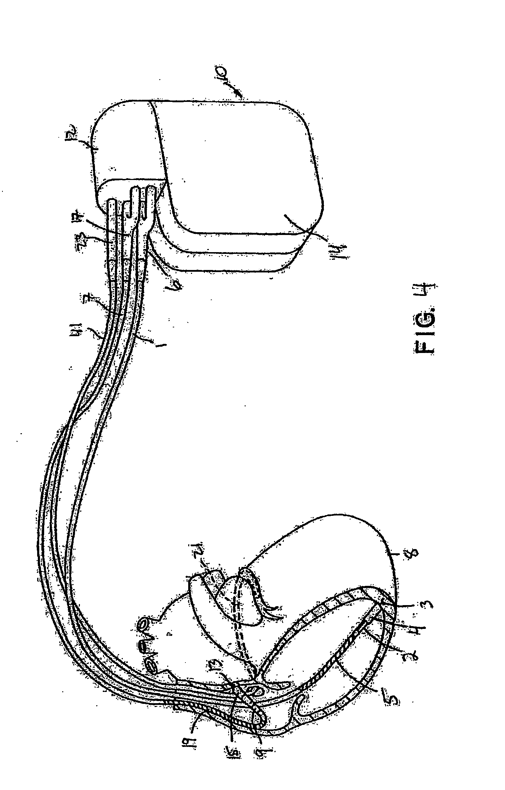

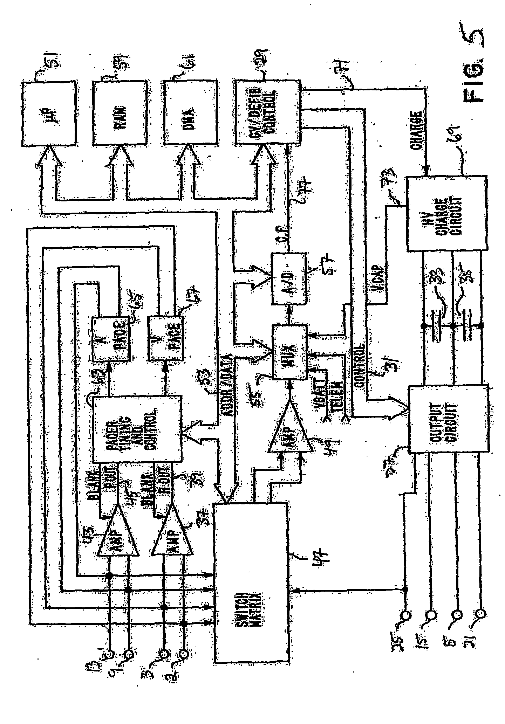

Figures 4 and 5 illustrate one embodiment of IMD 10 and a corresponding lead set, where IMD 10 is a PCD. In Figure 4, the ventricular lead takes the form of leads disclosed in U.S. Patent Nos. 5,099,838 and 5,314,430 to Bardy, and includes an elongated insulative lead body 1 carrying three concentric coiled conductors separated from one another by tubular insulative sheaths. Located adjacent the distal end of lead 1 are ring electrode 2, extendable helix electrode 3 mounted retractably within insulative electrode head 4 and elongated coil electrode 5. Each of the electrodes is coupled to one of the coiled conductors within lead body 1. Electrodes 2 and 3 are employed for cardiac pacing and for sensing ventricular depolarizations. At the proximal end of the lead is bifurcated connector 6 which carries three electrical connectors, each coupled to one of the coiled conductors. Defibrillation electrode 5 may be fabricated from platinum, platinum alloy or other materials known to be usable in implantable defibrillation electrodes and may be about 5 cm in length.

The atrial/SVC lead shown in Figure 4 includes elongated insulative lead body 7 carrying three concentric coiled conductors separated from one another by tubular insulative sheaths corresponding to the structure of the ventricular lead. Located adjacent the J-shaped distal end of the lead are ring electrode 9 and extendable helix electrode 13 mounted retractably within an insulative electrode head 15 Each of the electrodes is coupled to one of the coiled conductors within lead body 7. Electrodes 13 and 9 are employed for atrial pacing and for sensing atrial depolarizations. Elongated coil electrode 19 is provided proximal to electrode 9 and coupled to the third conductor within lead body 7. Electrode 19 preferably is 10 cm in length or greater and is configured to extend from the SVC toward the tricuspid valve. In one embodiment of the present invention, approximately 5 cm of the right atrium/SVC electrode is located in the right atrium with the remaining 5 cm located in the SVC. At the proximal end of the lead is bifurcated connector 17 carrying three electrical connectors, each coupled to one of the coiled conductors.

The coronary sinus lead shown in Figure 4 assumes the form of a coronary sinus lead disclosed in the above cited '838 patent issued to Bardy, and includes elongated insulative lead body 41 carrying one coiled conductor coupled to an elongated coiled defibrillation electrode 21. Electrode 21, illustrated in broken outline in Figure 4, is located within the coronary sinus and great vein of the heart. At the proximal end of the lead is connector plug 23 carrying an electrical connector coupled to the coiled conductor. The coronary sinus/great vein electrode 41 may be about 5 cm in length.

Implantable PCD 10 is shown in Figure 4 in combination with leads 1, 7 and 41, and lead connector assemblies 23, 17 and 6 inserted into connector block 12. Optionally, insulation of the outward facing portion of housing 14 of PCD 10 may be provided using a plastic coating such as parylene or silicone rubber, as is employed in some unipolar cardiac pacemakers. The outward facing portion, however, may be left uninsulated or some other division between insulated and uninsulated portions may be employed. The uninsulated portion of housing 14 serves as a subcutaneous defibrillation electrode to defibrillate either the atria or ventricles. Lead configurations other that those shown in Figure 4 may be practiced in conjunction with the present invention, such as those shown in U.S. Patent No. 5,690,686 to Min et al.

Figure 5 is a functional schematic diagram of one embodiment of implantable PCD 10 of the present invention. This diagram should be taken as exemplary of the type of device in which various embodiments of the present invention may be embodied, and not as limiting, as it is believed that the invention may be practiced in a wide variety of device implementations, including cardioverters and defibrillators.

IMD 10 is provided with an electrode system. If the electrode configuration of Figure 4 is employed, the correspondence to the illustrated electrodes is as follows. Electrode 25 in Figure 5 includes the uninsulated portion of the housing of PCD 10. Electrodes 25, 15, 21 and 5 are coupled to high voltage output circuit 27, which includes high voltage switches controlled by CV/defib control logic 29 via control bus 31. Switches disposed within circuit 27 determine which electrodes are employed and which electrodes are coupled to the positive and negative terminals of the capacitor bank (which includes capacitors 33 and 35) during delivery of defibrillation pulses.

Electrodes 2 and 3 are located on or in the ventricle and are coupled to the R-wave amplifier 37, which preferably takes the form of an automatic gain controlled amplifier providing an adjustable sensing threshold as a function of the measured R-wave amplitude. A signal is generated on R-out line 39 whenever the signal sensed between electrodes 2 and 3 exceeds the present sensing threshold.

Electrodes 9 and 13 are located on or in the atrium and are coupled to the P-wave amplifier 43, which preferably also takes the form of an automatic gain controlled amplifier providing an adjustable sensing threshold as a function of the measured P-wave amplitude. A signal is generated on P-out line 45 whenever the signal sensed between electrodes 9 and 13 exceeds the present sensing threshold. The general operation of R-wave and P-wave amplifiers 37 and 43 may correspond to that disclosed in U.S. Pat. No. 5,117,824, by Keimel et al., issued Jun. 2, 1992, for "An Apparatus for Monitoring Electrical Physiologic Signals"

Switch matrix 47 is used to select which of the available electrodes are coupled to wide band (0.5-200 Hz) amplifier 49 for use in digital signal analysis. Selection of electrodes is controlled by the microprocessor 51 via data/address bus 53, which selections may be varied as desired. Signals from the electrodes selected for coupling to bandpass amplifier 49 are provided to multiplexer 55, and thereafter converted to multi-bit digital signals by A/D converter 57, for storage in random access memory 59 under control of direct memory access circuit 61. Microprocessor 51 may employ digital signal analysis techniques to characterize the digitized signals stored in random access memory 59 to recognize and classify the patient's heart rhythm employing any of the numerous signal processing methodologies known to the art.

The remainder of the circuitry is dedicated to the provision of cardiac pacing, cardioversion and defibrillation therapies, and, for purposes of the present invention may correspond to circuitry known to those skilled in the art. The following exemplary apparatus is disclosed for accomplishing pacing, cardioversion and defibrillation functions. Pacer timing/control circuitry 63 preferably includes programmable digital counters which control the basic time intervals associated with DDD, WI, DVI, VDD, AAI, DDI and other modes of single and dual chamber pacing well known to the art. Circuitry 63 also preferably controls escape intervals associated with anti-tachyarrhythmia pacing in both the atrium and the ventricle, employing any anti-tachyarrhythmia pacing therapies known to the art.

Intervals defined by pacing circuitry 63 include atrial and ventricular pacing escape intervals, the refractory periods during which sensed P-waves and R-waves are ineffective to restart timing of the escape intervals and the pulse widths of the pacing pulses. The durations of these intervals are determined by microprocessor 51, in response to stored data in memory 59 and are communicated to pacing circuitry 63 via address/data bus 53. Pacer circuitry 63 also determines the amplitude of the cardiac pacing pulses under control of microprocessor 51.

During pacing, escape interval counters within pacer timing/control circuitry 63 are reset upon sensing of R-waves and P-waves as indicated by a signals on lines 39 and 45, and in accordance with the selected mode of pacing on time-out trigger generation of pacing pulses by pacer output circuitry 65 and 67, which are coupled to electrodes 9, 13, 2 and 3. Escape interval counters are also reset on generation of pacing pulses and thereby control the basic timing of cardiac pacing functions, including anti-tachyarrhythmia pacing. The durations of the intervals defined by escape interval timers are determined by microprocessor 51 via data/address bus 53. The value of the count present in the escape interval counters when reset by sensed R-waves and P-waves may be used to measure the durations of R-R intervals, P-P intervals, P-R intervals and R-P intervals, which measurements are stored in memory 59 and used to detect the presence of tachyarrhythmias.

Microprocessor 51 most preferably operates as an interrupt driven device, and is responsive to interrupts from pacer timing/control circuitry 63 corresponding to the occurrence of sensed P-waves and R-waves and corresponding to the generation of cardiac pacing pulses. Those interrupts are provided via data/address bus 53. Any necessary mathematical calculations to be performed by microprocessor 51 and any updating of the values or intervals controlled by pacer timing/control circuitry 63 take place following such interrupts.

Detection of atrial or ventricular tachyarrhythmias, as employed in the present invention, may correspond to tachyarrhythmia detection algorithms known in the art. For example, the presence of an atrial or ventricular tachyarrhythmia may be confirmed by detecting a sustained series of short R-R or P-P intervals of an average rate indicative of tachyarrhythmia or an unbroken series of short R-R or P-P intervals. The suddenness of onset of the detected high rates, the stability of the high rates, and a number of other factors known in the art may also be measured at this time. Appropriate ventricular tachyarrhythmia detection methodologies measuring such factors are described in U.S. Pat. No. 4,726,380 issued to Vollmann, U.S. Pat. No. 4,880,005 issued to Pless et al. and U.S. Pat. No. 4,830,006 issued to Haluska et al.

An additional set of tachycardia recognition methodologies is disclosed in the article "Onset and Stability for Ventricular Tachyarrhythmia Detection in an Implantable Pacer-Cardioverter-Defibrillator" by Olson et al., published in Computers in Cardiology, Oct. 7-10, 1986, IEEE Computer Society Press, pages 167-170. Atrial fibrillation detection methodologies are disclosed in Published PCT Application Ser. No. US92/02829, Publication No. WO92/18198, by Adams et al., and in the article "Automatic Tachycardia Recognition", by Arzbaecher et al., published in PACE, May-June, 1984, pp. 541-547.

In the event an atrial or ventricular tachyarrhythmia is detected and an anti-tachyarrhythmia pacing regimen is desired, appropriate timing intervals for controlling generation of anti-tachyarrhythmia pacing therapies are loaded from microprocessor 51 into the pacer timing and control circuitry 63, to control the operation of the escape interval counters therein and to define refractory periods during which detection of R-waves and P-waves is ineffective to restart the escape interval counters.

Alternatively, circuitry for controlling the timing and generation of anti-tachycardia pacing pulses as described in U.S. Pat. No. 4,577,633, issued to Berkovits et al. on Mar. 25, 1986, U.S. Pat. No. 4,880,005, issued to Pless et al. on Nov. 14, 1989, U.S. Pat. No. 4,726,380, issued to Vollmann et al. on Feb. 23, 1988 and U.S. Pat. No. 4,587,970, issued to Holley et al. on May 13, 1986, may also be employed.

In the event that generation of a cardioversion or defibrillation pulse is required, microprocessor 51 may employ an escape interval counter to control timing of such cardioversion and defibrillation pulses, as well as associated refractory periods. In response to the detection of atrial or ventricular fibrillation or tachyarrhythmia requiring a cardioversion pulse, microprocessor 51 activates cardioversion/defibrillation control circuitry 29, which initiates charging of the high voltage capacitors 33 and 35 via charging circuit 69, under the control of high voltage charging control line 71. The voltage on the high voltage capacitors is monitored via VCAP line 73, which is passed through multiplexer 55 and in response to reaching a predetermined value set by microprocessor 51, results in generation of a logic signal on Cap Full (CF) line 77 to terminate charging. Thereafter, timing of the delivery of the defibrillation or cardioversion pulse is controlled by pacer timing/control circuitry 63. Following delivery of the fibrillation or tachycardia therapy microprocessor 51 returns the device to a cardiac pacing mode and awaits the next successive interrupt due to pacing or the occurrence of a sensed atrial or ventricular depolarization.

Several embodiments of appropriate systems for the delivery and synchronization of ventricular cardioversion and defibrillation pulses and for controlling the timing functions related to them are disclosed in U.S. Patent No. 5,188,105 to Keimel, U.S. Pat. No. 5,269,298 to Adams et al. and U.S. Pat. No. 4,316,472 to Mirowski et al.

Any known cardioversion or defibrillation pulse control circuitry is believed to be usable in conjunction with various embodiments of the present invention, however. For example, circuitry controlling the timing and generation of cardioversion and defibrillation pulses such as that disclosed in U.S. Patent No. 4,384,585 to Zipes, U.S. Patent No. 4,949,719 to Pless et al., or U.S. Patent No. 4,375,817 to Engle et al..

Continuing to refer to Figure 5, delivery of cardioversion or defibrillation pulses is accomplished by output circuit 27 under the control of control circuitry 29 via control bus 31. Output circuit 27 determines whether a monophasic or biphasic pulse is delivered, the polarity of the electrodes and which electrodes are involved in delivery of the pulse. Output circuit 27 also includes high voltage switches which control whether electrodes are coupled together during delivery of the pulse. Alternatively, electrodes intended to be coupled together during the pulse may simply be permanently coupled to one another, either exterior to or interior of the device housing, and polarity may similarly be pre-set, as in current implantable defibrillators. An example of output circuitry for delivery of biphasic pulse regimens to multiple electrode systems may be found in the above cited patent issued to Mehra and in U.S. Patent No. 4,727,877

An example of circuitry which may be used to control delivery of monophasic pulses is disclosed in U.S. Patent No. 5,163,427 to Keimel,
Output control circuitry similar to that disclosed in U.S. Patent No. 4,953,551 to Mehra et al. or U.S. Patent No. 4,800,883 to Winstrom,
may also be used in conjunction with various embodiments of the present invention to deliver biphasic pulses.

Alternatively, IMD 10 may be an implantable nerve stimulator or muscle stimulator such as that disclosed in U.S. Patent No. 5,199,428 to Obel et al., U.S. Patent No. 5,207,218 to Carpentier et al. or U.S. Patent No. 5,330,507 to Schwartz, or an implantable monitoring device such as that disclosed in U.S. Patent No. 5,331,966 issued to Bennet et al.,

The present invention is believed to find wide application to any form of implantable electrical device for use in conjunction with electrical leads.

In practicing the method of predicting AF according to this invention a lead 16 having a tip electrode 21 is positioned in the right atrium so that the tip electrode is in place against the interior atrial wall, in a conventional manner. This is illustrated in Figure 7A, as described above. It is noted that there is no criticality as to exactly where the tip electrode is placed. The lead may be a unipolar lead, in which case the pacemaker case is used as the other electrode; or the lead may have one or more other electrodes, with an electrode displaced significantly proximal to the tip electrode being used as the other electrode so that sensing is effectively unipolar.

Our studies have shown that during normal sinus rhythm the conduction velocity is substantially homogeneous throughout the entire atria. What we want to measure is the deviation in normal conduction velocity, which is substantially the same for the whole atria. The local sensing performed by the tip electrode obtains a signal that is representative of conduction velocity throughout the atria.

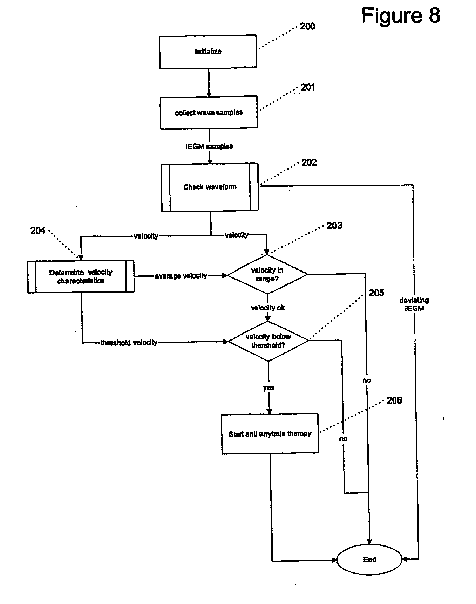

Figure 8 presents a flow diagram that shows the steps taken in the practice of this invention to monitor conduction velocity. The preferred use is for measuring atrial conduction, in order to detect the onset of AF. However, the same algorithm can be used to detect changes in the right ventricle, using IECG signals sensed from passing ventricular excitation waves (R waves). At 200, the algorithm is initiated. The specific AF prediction algorithm of this invention need not be performed every cycle, but only periodically, e.g., every tenth beat. Changes in conduction velocity may occur only over relatively long time periods, such that monitoring conduction velocity for AF need not be done too frequently. However, it is important to detect any change as quickly as possible so as to provide timely therapy. Consequently, the time period for initiating the prediction algorithm of Figure 8 is a matter of choice, and may be subject to knowledge of the patient's past history. Also at 200 the pacemaker checks to see that conditions are appropriate for the test, e.g., the atrial rate is nominal and stable. If rate is not stable, then changes in the width of the P-wave might not suggest changes in conduction velocity.

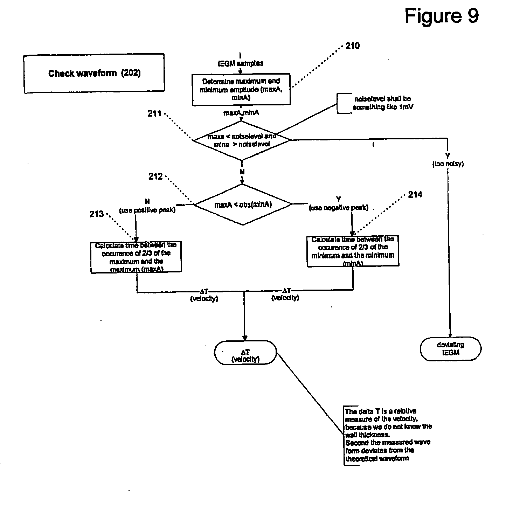

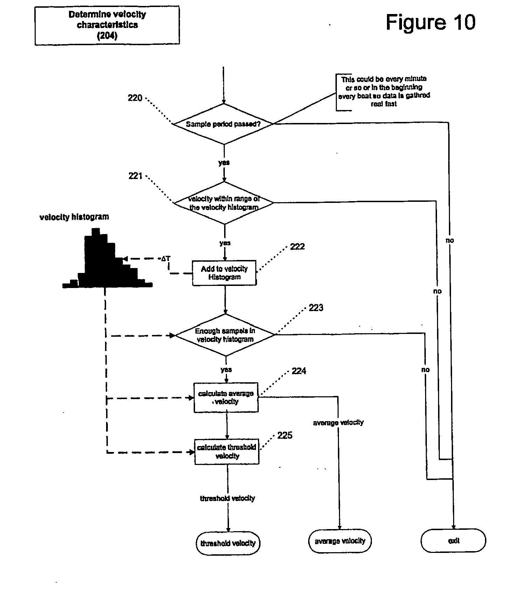

As the excitation wave depicted in Figure 6 advances over the entire atria it proceeds from the sinus node through to the last activated area located below the inferior pulmonary vein on the left lateral wall. For normal pacemaker operation, the P-wave is detected and processed every cycle, for control purposes. Samples of the sensed excitation wave, or intracardial P-wave are sensed and collected as shown at block 201. Generally, a plurality of P-wave samples is taken at each monitoring episode. For example, n consecutive IEGM (P-wave) samples may be collected for the purpose of the AF prediction algorithm. At 202, pertinent P-wave characteristics are determined. In the embodiment of Figure 8, the maximum and minimum amplitudes are determined by checking the waveform. In a preferred embodiment, the duration is determined as the Δt from 2/3 of maximum amplitude of the rising slope to that of the falling wave slope, as discussed in more detail below in connection with Figure 9. Other formulae can be employed to obtain a measure of the wave duration Δt within the scope of the invention. The morphology of the P-waves may also be checked, to determine that they reflect normal physiological events. If the waveforms are found to be deviating, the samples must be rejected, and the routine exits. However, if they are nominal, then the routine continues. The "velocity" measure that is provided from the operation at 202 is a relative velocity, as the atrial wall thickness is not known. Further, the waveform will deviate somewhat from the theoretical. At 204, the velocity samples from the operation at 202 are stored, and a subroutine (Figure 10) is employed to obtain certain velocity characteristics. The wave widths, representing relative velocity values, are suitably averaged to obtain a value V representative of average conduction velocity for the patient. Additionally, a velocity threshold is calculated, for subsequent use in determining whether a change in conduction velocity is indicative of AF (or VF, in a ventricular embodiment).

At block 203, the routine compares the current velocity with the average velocity to determine if it is within a proper range. If the current velocity reading deviates significantly from the prior range, the data is considered not good, and the routine exits. However, if the velocity measure is ok, then the velocity is compared to the threshold velocity at 205. If velocity V is not below the threshold, the routine concludes that AF is not predicted, and exits. If velocity is below threshold, then at 206 the system initiates a suitable anti-arrhythmia therapy.

Figure 9 illustrates a suitable "check waveform" subroutine, corresponding to block 202 of Figure 8. At block 210, the maximum (positive) and minimum (negative) amplitudes are determined for each sensed waveform. The values, shown as maxA and minA respectively, are compared at 211 to a predetermined noise level. Noise level may be set at, for example, about 1 mV. If neither amplitude is greater than the noise level, the conclusion is that the data is too noisy, and the routine exits. However, if this is not the case, then at 212 the routine compares maxA and the absolute value of minA, and picks the largest of the two values, i.e., determines whether to use the positive or negative peak. For the positive peak, at 213 Δt is calculated as the time between 2/3 of the maximum of the rising slope and 2/3 of the maximum of the falling slope. Of course, other criteria for obtaining a measure of the duration value Δt can be employed within the scope of the invention, the specific measure being a matter of choice. Likewise, at 214 a value of Δt is obtained from the negative peak, if the negative peak has been chosen. In either event, the product of the subroutine is a value of Δt for each analyzed waveform, which value represents relative velocity for the conduction velocity embodiment of this invention.

Figure 10 provides a flow diagram of block 204, detailing steps taken to determine certain velocity characteristics for the velocity determination embodiments. At 220, it is determined whether a predetermined sample period has passed. The sample period is a variable, and could be, e.g., every minute. However, at start up of the pacemaker system, this could be set to operate on every beat, so as to build up a database quickly. If the sample period has passed, the routine then goes to step 221 and determines whether the velocity of each waveform is within the range of a stored velocity histogram, i.e., whether it is a value that is reasonable and can be accepted. If the velocity data is not within the range, the routine exits; if yes, it goes to block 222 and adds each sample to a velocity histogram that is continually built in memory. At 223, the routine determines whether there are enough samples in the velocity histogram to provide a good statistical basis. Thus, at start up following implant, or when turning on the embodiment by external programming, it is necessary to wait to get a good database. When this has been accomplished, at 224 the routine calculates an average (relative) velocity. Then, at 225, a threshold velocity is calculated. This may be done just once, when the histogram has sufficient samples; or it may be repeated a predetermined number of times following start up. Threshold is a figure that optionally may be initially programmed by the physician. However, it preferably is calculated as a function of initial average velocity. For example, threshold may be calculated as Vavg + X% of Vavg, where X is a programmable value in the range of 20-50. Other methods of determining the threshold value may be used within the scope of the invention.

It is noted that determination of onset of AF can be done in other ways than as depicted and described in connection with block 205 in Figure 8. Thus, rather than calculating a threshold, the algorithm may look for a predetermined decrease in the measure of velocity over a predetermined relatively long time period, e.g., a 25% decrease over a month. Further, depending on patient history, the response may be to simply post a flag that indicates onset of AF, so that at a subsequent confirmation an AF therapy will be commenced. Or, the response can be to immediately initiate a relatively high rate pacing sequence to attempt to break up the dangerous condition. Anti-AF pacing therapies are well known in the art, and the invention embraces any suitable such pacing therapy.

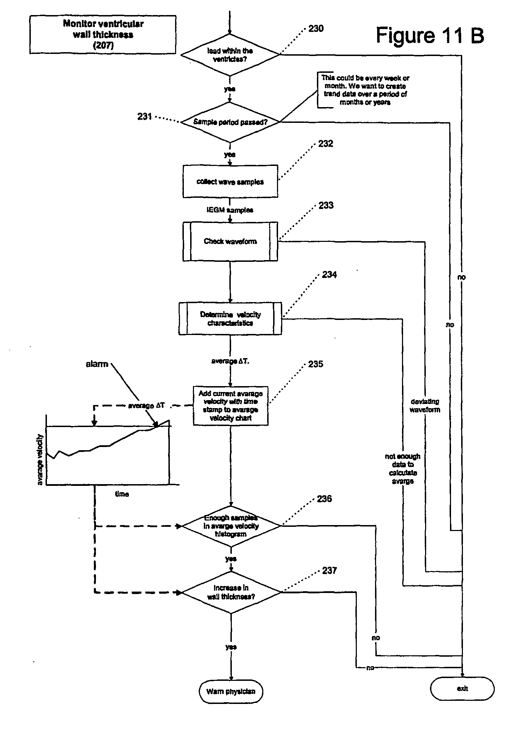

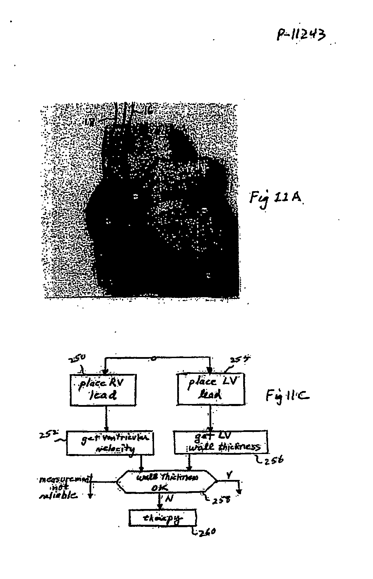

The embodiment of this invention for determining ventricular wall thickness is shown in Figures 11A,B and C. Figure 11A illustrates the placement of a lead 150 having a tip electrode 151 that is positioned adjacent to the left free ventricular wall. The lead is passed through the coronary sinus to an epicardial vein on the free wall of the left ventricle, in the same manner as is done for bi-ventricular pacing systems. The tip 151 detects the repolarization wave (R wave) as it passes by the tip, in the same manner as described above for the atrial wave. The pacemaker case is suitably used as the indifferent electrode. A second electrode for pacing may be positioned in normal fashion in the right ventricle, substantially at the apex. The R-waves sensed by the left ventricular tip electrode (RL) are processed in the same manner as done in the AF predictor algorithm. Concurrently, R-waves sensed by the right ventricular electrode (RR) are examined to verify that overall there has been no significant change in ventricular conduction velocity. Having verified this, then the changes in width (Δt) of the wave sensed by the left ventricular electrode can be interpreted as changes in ventricular wall width. As discussed above and as illustrated in Figure 11B, the longer the wave duration (Δt) the greater the wall thickness. In this embodiment, changing values of relative wall thickness are compared with a wall threshold value, and appropriate pacing therapy or an adjustment in medication is initiated when the threshold is exceeded.

Figure 11B shows an exemplary flow diagram of an algorithm for determining the wall thickness of the free wall of the left ventricle. Such determination can be very useful for assessing hypertrophy of the ventricle, so that appropriate therapy can be undertaken. As discussed above, measurement of ventricular wall thickness is undertaken with the assumption that conduction velocity in the left ventricle is substantially constant, and that a significant increase in the width (Δt) of the sensed R wave is due to enlargement of the left wall thickness.

As illustrated in Figure 11B, at 230 it is determined that a lead is properly placed within the left ventricle or adjacent to the left ventricular wall (as illustrated in Figure 11C). Assuming that this condition is met, at 231 the routine tests to see that a programmable sample period has passed. If not, the routine exits, but if yes, it goes on to block 232 and collects wave samples in the same manner as discussed in connection with the routine of Figure 8. The IEGM samples are checked for waveform at 233, according to the subroutine disclosed in Figure 9, yielding representations of Δt that, in this case, are measures of relative left ventricular wall thickness. The steps taken in block 233 may also include a check to determine that the wall thickness measure is within an acceptable range, corresponding to step 203 in Figure 8. The processing at block 233 produces duration measures that are sent for processing at block 234. Block 234 performs the same operations as shown in Figure 10, i.e., the same "determine velocity characteristics" operations are carried out. However, in this embodiment the Δt values represent relative left ventricular wall thickness and values of average Δt are calculated. At 235 the average Δtavg values are added, along with a time stamp, to a histogram in the same manner as illustrated for the velocity data in Figure 10. At 236, the routine determines whether enough samples have been placed in the histogram. If not, the routine exits until a sufficient database has been built. If there is enough data, then at 237 the routine determines whether or not there has been a significant increase in left ventricular wall thickness. This may be done in a chosen one of several ways. For example, the average Δt value may be compared to a predetermined alarm value and if it exceeds the alarm value, then a warning is posted to the physician. The warning can be in any suitable form, such as by setting a flag that is read whenever the physician next checks the device. Alternatively, the determination can be made by looking for a given percentage increase in relative wall thickness over a period of time, e.g., a relatively long period of time such as a 1-6 months, or even annually. Since wall thickness does not change rapidly, this algorithm need only be entered once a week, for example. Further, the physician will be interested in long term trend data.

In both this embodiment and the atrial velocity embodiment, heart rate may be checked to make sure that it is stable, and if not stable the algorithm exits. Another embodiment is illustrated by the flow diagram of Figure 11C. In this embodiment, RR waves are collected from the right ventricle and used to determine whether ventricular conduction velocity has remained substantially constant. This is done by examining the Δt of sensed RR waves in the same manner as described above for atrial waves, to ensure that the right R wave signal has maintained a substantially constant duration. A substantially constant duration of RR waves provides confirmation of stable ventricular conduction velocity, such that changes in the measurement of wave duration at the left ventricular wall can be reliably taken as indicating change in wall thickness.

As shown in Figure 11C, at 250 a lead 18 is placed in the right ventricle, having a tip electrode 29. For purposes of measuring conduction velocity, the tip electrode may be positioned other than at the apex. At 252, a measure of ventricular conduction velocity is obtained, using the same flow diagram as in Figure 8; the velocity threshold is set to indicate whether or not there has been a significant change in velocity. A lead 150 is placed so that the tip 151 is adjacent the free wall of the left ventricle, as indicated at block 254. At 256, the left ventricular wall thickness is obtained, as per Figure 11B. At 258, the algorithm checks the ventricular velocity to determine if it is stable. If not, the routine exits, as the wall thickness measure is then not accurate. If velocity is stable, then the routine determines if the wall thickness is OK, e.g., has the increase exceeded a predetermined limit? If wall thickness is OK, the routine exits; if not, then therapy is applied, as indicated at 260.

It is noted that the invention can be practiced with various combinations of determining conduction velocity and wall thickness. As illustrated in Figure 11A, the invention comprises a multi-chamber embodiment, where either or both ventricular and atrial conduction velocity can be determined, and left ventricular wall thickness can be determined. The choice of functions can be programmable, in accord with the current state of the art.

The present invention is not limited to any particular combination of hardware and software per se, but may find further application with any form of software supplementing hardware. For example, other software embodiments that achieve the ability to efficiently store and manipulate the data, and analyze the sensed depolarization signals, are within the scope of the invention.

## Claims

1. A system for continuously determining a characteristic of a depolarization wave in a wall of a patient's heart and for initiating a pacing response to the heart as a function of said determining, comprising:
signal means for obtaining a signal representative of the passage of a depolarization wave past a point on said wall;
Δt means for determining a measure of the signal width of the depolarization wave from each said obtained signal;
analyzing means for analyzing said signal width measures, **characterised by**
said signal means comprising a tip electrode to be positioned adjacent to the patient's left free ventricular wall, said and analyzing means comprising wall thickness means for determining from said signal width measures the patient's left free ventricular wall thickness, said and wall thickness means comprising criteria means for comparing said signal width measures with predetermined width criteria and for indicating an increase in wall thickness indicative of ventricular tachyarrhythmia if said signal width measures exceed a predetermined increase in width; and
response means for initiating a pacing therapy in response to said predetermined increase

2. The system as described in claim 1, further wherein said signal means comprises a tip electrode to be positioned against the right atrial wall of said heart; said analyzing means comprises velocity means for determining conduction velocity in said heart; and said velocity means comprises criteria means for comparing said signal width measures with predetermined width criteria and for indicating a reduction in conduction velocity indicative of atrial fibrillation if said signal width measures exceed said predetermined increase

3. The system as described in claim 1 or 2, wherein said signal means further comprises stability means for determining whether said signals are stable.

4. The system as described in claim 3, comprising rate means for limiting the operation of said system to conditions where the cardiac rate is within a predetermined range.

5. The system as described in claim 1 or 2, comprising threshold means for determining a velocity threshold, comparison means for comparing said conduction velocity with said threshold, and wherein said response means is enabled when conduction velocity is determined to be slower than said threshold.

6. The system as described in claim 5, wherein said threshold means comprises set means for setting said threshold as a function of signal width measures.

7. The system as described in claim 6, wherein said threshold means comprises programmable threshold criteria.

8. A system as claimed in claim 1 or 2 for predicting the onset of an atrial arrhythmia, wherein said tip electrode is adapted to be positioned against the myocardial wall of the patient's right atrium; and wherein said depolarization wave is a P-wave; and wherein said response means responds by providing a predetermined AF (atrial fibrillation) pacing therapy.

9. The system as described in claim 8, wherein said processing means comprises determining means for operating on a P-wave to determine the signal width in milliseconds between two predetermined reference points on said P-wave.

10. The system as described in claim 9, wherein said determining means comprises amplitude means for determining the maximum amplitude of a processed P-wave signal and reference means for setting said reference points relative to said maximum amplitude.

11. The system as described in claim 10, wherein said reference means comprises means for setting said reference points where the leading and trailing edges of said P-wave are a predetermined fraction below said maximum amplitude.

12. The system as described in claim 10, wherein said amplitude means further comprises minimum means for determining the minimum amplitude of a processed P-wave, and said reference means comprises means for setting said reference points where the leading and trailing edges of said P-wave are a predetermined distance between said maximum and minimum amplitudes.

13. The system as described in claim 1 or 2, comprising storage means for storing data representative of said width measures over said long period.

14. The system as described in claim 1 or 2, comprising checking means for checking the reliability of said wave samples in terms of indicating relative conduction velocity.

15. The system as described in claim 14, wherein said checking means comprises noise comparison means for determining that said waveforms are of significant amplitude above the prevailing noise level.

16. The system as described in claim 15, wherein said checking means comprises rate means for determining that the rate of said wave samples is within a predetermined range.

17. The system as described in claim 1 or 2, comprising sample means for determining that the collected number of waveform samples is sufficient to provide an accurate result.

## Patentansprüche

1. System zum kontinuierlichen Bestimmen eines Charakteristikums bzw. einer Eigenschaft einer Depolarisationswelle in einer Wand eines Herzens eines Patienten und zum Einleiten einer Stimulationsantwort an das Herz als einer Funktion des Bestimmens, mit:
Signalmitteln zum Erhalten eines Signals, das für die Passage einer Depolarisationswelle an einem Punkt auf der Wand vorbei repräsentativ bzw. kennzeichnend ist,
Δt-Mitteln zum Bestimmen eines Maßes der Signalbreite der Depolarisationswelle aus jedem erhaltenen Signal;
Analysemitteln zum Analysieren der Signalbreitenmaße, **dadurch gekennzeichnet, dass**
die Signalmittel eine Spitzenelektrode aufweisen, die neben der linken freien ventrikulären Wand bzw. der Wand des linken freien Ventrikels des Patienten zu positionieren ist, und
die Analysemittel Wanddickemittel zum Bestimmen der Wanddicke der linken freien ventrikulären Wand des Patienten aus den Signalbreitemaßen aufweisen, und
die Wanddickemittel Kriterienmittel zum Vergleichen der Signalbreitenmaße mit vorbestimmten Breitenkriterien und zum Anzeigen einer Zunahme der Wanddicke, die kennzeichnend für ventrikuläre Tachyarrhytmie ist, wenn die Signalbreitenmaße eine vorbestimmte Zunahme in der Breite übersteigen, aufweisen; und
Antwortmittel zum Einleiten einer Stimulationstherapie als Antwort auf die vorbestimmte Zunahme vorgesehen sind.

2. System nach Anspruch 1, bei dem ferner die Signalmittel eine Spitzenelektrode zur Positionierung auf der rechten atrialen Wand des Herzens aufweisen; die Analysemittel Geschwindigkeitsmittel zum Bestimmen der Leitungsgeschwindigkeit in dem Herzen aufweisen; und die Geschwindigkeitsmittel Kriterienmittel zum Vergleichen der Signalbreitenmaße mit vorbestimmten Breitenkriterien und zum Anzeigen einer Verringerung der Leitungsgeschwindigkeit, die kennzeichnend für eine atriale Fibrillation ist, wenn die Signalbreitenmaße die vorbestimmte Zunahme übersteigen, aufweisen.

3. System nach Anspruch 1 oder 2, bei dem die Signalmittel ferner Stabilitätsmittel zum Bestimmen, ob die Signale stabil sind, aufweisen.

4. System nach Anspruch 3, das Ratenmittel zum Beschränken des Betriebs auf Bedingungen, in denen die Herzrate bzw. -frequenz innerhalb eines vorbestimmten Bereichs ist bzw. liegt, aufweist.

5. System nach Anspruch 1 oder 2, das Grenzwertmittel zum Bestimmen eines Geschwindigkeitsgrenzwertes, Vergleichsmittel zum Vergleichen der Leitungsgeschwindigkeit mit dem Grenzwert aufweist, und bei dem die Antwortmittel freigegeben werden, wenn bestimmt wird, dass die Leitungsgeschwindigkeit langsamer als der Grenzwert ist.

6. System nach Anspruch 5, bei dem die Grenzwertmittel Setz- bzw. Einstellmittel zum Setzen bzw. Einstellen des Grenzwerts als einer Funktion der Signalbreitenmaße aufweisen.

7. System nach Anspruch 6, bei dem die Grenzwertmittel programmierbare Grenzwertkriterien aufweisen.

8. System nach Anspruch 1 oder 2 zum Vorhersagen des Einsetzens einer atrialen Arrhythmie, bei dem die Spitzenelektrode dafür eingerichtet ist, auf der myokardialen Wand des rechten Atriums des Patienten positioniert zu werden; und bei dem die Depolarisationswelle eine P-Welle ist; und bei dem die Antwortmittel durch Bereitstellen einer vorbestimmten AF (atrialen Fibrillations-) Stimulationstherapie antworten.

9. System nach Anspruch 8, bei dem die Verarbeitungsmittel Bestimmungsmittel zum Einwirken auf eine bzw. zum Arbeiten mit einer P-Welle zum Bestimmen der Signalbreite in Millisekunden zwischen zwei vorbestimmten Referenz- bzw. Bezugspunkten auf der P-Welle aufweisen.

10. System nach Anspruch 9, bei dem die Bestimmungsmittel Amplitudenmittel zum Bestimmen der maximalen Amplitude eines verarbeiteten P-Wellensignals und Referenzmittel zum Setzen der Referenzpunkte in Bezug auf die maximale Amplitude aufweisen.

11. System nach Anspruch 10, bei dem die Referenzmittel Mittel zum Setzen der Referenzpunkte aufweisen, wobei die Anstiegs- und Abfallflanken bzw. -kanten der P-Welle in einem vorbestimmten Anteil bzw. ein vorbestimmter Anteil unter der maximalen Amplitude sind bzw. liegen.

12. System nach Anspruch 10, bei dem die Amplitudenmittel ferner Minimum- bzw. Minimalmittel zur Bestimmung der minimalen Amplitude einer verarbeiteten P-Welle aufweisen, und die Referenzmittel Mittel zum Setzen der Referenzpunkte aufweisen, wobei die Anstiegs- und Abfallflanken der P-Welle in einer vorbestimmte Distanz zwischen den maximalen und minimalen Amplituden liegen.

13. System nach Anspruch 1 oder 2, das Speichermittel zum Speichern von Daten, die repräsentativ bezüglich der Breitenmaße über den langen Zeitraum sind.

14. System nach Anspruch 1 oder 2, das Prüfmittel zum Prüfen der Verlässlichkeit der Wellentestwerte im Sinne des Anzeigens relativer Leitgeschwindigkeit aufweisen.

15. System nach Anspruch 14, bei dem die Prüfmittel Rauschvergleichsmittel zum Bestimmen, dass die Wellenformen eine signifikante Amplitude oberhalb des vorherrschenden Rauschniveaus besitzen, aufweisen.

16. System nach Anspruch 15, bei dem die Prüfmittel Ratenmittel zum Bestimmen, dass die Rate der Wellentestwerte innerhalb eines vorbestimmten Bereichs ist, aufweisen.

17. System nach Anspruch 1 oder 2, das Testwertmittel zum Bestimmen, dass die gesammelte Anzahl von Wellenformtestwerten ausreichend zur Bereitstellung eines genauen Ergebnisses ist, aufweist.

## Revendications

1. Système pour déterminer en continu une caractéristique d'une onde de dépolarisation dans une paroi d'un coeur de patient et pour déclencher une réponse de stimulation du coeur en fonction de ladite détermination, comportant :
des moyens de signalisation pour obtenir un signal représentatif du passage d'une onde de dépolarisation devant un point sur ladite paroi ;
des moyens Δt pour déterminer une mesure de la largeur de signal de l'onde de dépolarisation à partir de chaque dit signal obtenu ;
des moyens d'analyse pour analyser lesdites mesures de largeur de signal, **caractérisé en ce que**
lesdits moyens de signalisation comportent une électrode d'embout devant être positionnée adjacente à la paroi ventriculaire libre gauche du patient, et
lesdits moyens d'analyse comportent des moyens d'épaisseur de paroi pour déterminer à partir desdites mesures de largeur de signal l'épaisseur de la paroi ventriculaire libre gauche du patient, et
lesdits moyens d'épaisseur de paroi comportent des moyens de comparaison de critères pour comparer lesdites mesures de largeur de signal à des critères de largeur prédéterminés et pour indiquer une augmentation d'épaisseur de paroi indicative d'une tachyarythmie ventriculaire si lesdites mesures de largeur de signal dépassent une augmentation prédéterminée de la largeur ; et
qu'il comporte des moyens de réponse pour déclencher une thérapie de stimulation en réponse à ladite augmentation prédéterminée.

2. Système tel que décrit dans la revendication 1, dans lequel lesdits moyens de signalisation comportent également une électrode d'embout devant être positionnée contre la paroi auriculaire droite dudit coeur ; lesdits moyens d'analyse comportent des moyens de vitesse pour déterminer une vitesse de conduction dans ledit coeur ; et lesdits moyens de vitesse comportent des moyens de comparaison de critères pour comparer lesdites mesures de largeur de signal à des critères de largeur prédéterminés et pour indiquer une réduction de la vitesse de conduction indicative d'une fibrillation auriculaire si lesdites mesures de largeur de signal dépassent ladite augmentation prédéterminée.

3. Système tel que décrit dans la revendication 1 ou 2, dans lequel lesdits moyens de signalisation comportent en outre des moyens de détermination de stabilité pour déterminer si lesdits signaux sont stables.

4. Système tel que décrit dans la revendication 3, comportant des moyens de fréquence ou taux pour limiter le fonctionnement dudit système à des états où la fréquence ou taux cardiaque est dans une plage prédéterminée.

5. Système tel que décrit dans la revendication 1 ou 2, comportant des moyens de seuil pour déterminer un seuil de vitesse, des moyens de comparaison pour comparer ladite vitesse de conduction audit seuil, et dans lequel lesdits moyens de réponse sont activés lorsqu'on détermine qu'une vitesse de conduction est inférieure audit seuil.

6. Système tel que décrit dans la revendication 5, dans lequel lesdits moyens de seuil comportent des moyens de réglage pour établir ledit seuil en fonction de mesures de largeur de signal.

7. Système tel que décrit dans la revendication 6, dans lesquels lesdits moyens de seuil comportent des critères de seuil programmables.

8. Système tel que revendiqué dans la revendication 1 ou 2 pour prédire le début d'une arythmie auriculaire, dans lequel ladite électrode d'embout est adaptée pour être positionnée contre la paroi myocardique de l'oreillette droite du patient ; et dans lequel ladite onde de dépolarisation est une onde P ; et dans lequel lesdits moyens de réponse répondent en délivrant une thérapie de stimulation AF (fibrillation auriculaire) prédéterminée.

9. Système tel que décrit dans la revendication 8, dans lequel lesdits moyens de traitement comportent des moyens de détermination pour un fonctionnement ou une opération sur une onde P pour déterminer la largeur de signal en millise-condes entre deux points de référence prédéterminés sur ladite onde P.

10. Système tel que décrit dans la revendication 9, dans lequel lesdits moyens de détermination comportent des moyens de mesure d'amplitude pour déterminer l'amplitude maximale d'un signal d'onde P traité et des moyens de référence pour établir lesdits points de référence par rapport à ladite amplitude maximale.

11. Système tel que décrit dans la revendication 10, dans lequel lesdits moyens de référence comportent des moyens pour établir lesdits points de référence où les flancs avant et arrière de ladite onde P sont une fraction prédéterminée sous ladite amplitude maximale.

12. Système tel que décrit dans la revendication 10, dans lequel lesdits moyens d'amplitude comportent également des moyens d'un minimum pour déterminer l'amplitude minimale d'une onde P traitée, et lesdits moyens de référence comportent des moyens pour établir lesdits points de référence où les flancs avant et arrière de ladite onde P sont à une distance prédéterminée entre lesdites amplitudes maximale et minimale.

13. Système tel que décrit dans la revendication 1 ou 2, comportant des moyens de mémorisation pour mémoriser des données représentatives desdites mesures de largeur sur ladite longue période.

14. Système tel que décrit dans la revendication 1 ou 2, comportant des moyens de contrôle pour contrôler la fiabilité desdits échantillons d'onde en termes d'indication de vitesse de conduction relative.

15. Système tel que décrit dans la revendication 14, dans lequel lesdits moyens de contrôle comportent des moyens de comparaison de bruit pour déterminer que lesdites formes d'onde ont une amplitude significative au-dessus du niveau de bruit dominant.

16. Système tel que décrit dans la revendication 15, dans lequel lesdits moyens de contrôle comportent des moyens de fréquence ou taux pour déterminer que la fréquence desdits échantillons d'onde est dans une plage prédéterminée.

17. Système tel que décrit dans la revendication 1 ou 2, comportant des moyens d'échantillonnage pour déterminer que le nombre collecté d'échantillons de forme d'onde est suffisant pour fournir un résultat précis.
